# EUROPEAN PATENT SPECIFICATION

(11) **EP 3 909 535 B1**
(45) Date of publication and mention of the grant of the patent: **03.07.2024**
(21) Application number: 21177886.5
(22) Date of filing: 11.03.2014
(51) Int. Cl.: A61B 18/14, A61B 18/00, A61B 90/00

(54) **ELECTROSURGICAL DEVICE HAVING A DISTAL APERTURE**
ELEKTROCHIRURGISCHE VORRICHTUNG MIT EINER DISTALEN APERTUR
DISPOSITIF ÉLECTROCHIRURGICAL AYANT UNE OUVERTURE DISTALE

(30) Priority: 15.03.2013 US 201361787617 P
(43) Date of publication of application: 17.11.2021
(62) Divisional of application: 19167716.0
(73) Proprietor: Boston Scientific Medical Device Limited, Galway H91 Y868 (IE)
(72) Inventor: DAVIES, Gareth, Toronto, M5R 2N5 (CA); URBANSKI, John Paul, Toronto, M4L 2N7 (CA); HARFIELD, Ellen, Mississauga, L4Z 3W8 (CA); MIRZA, Mahmood, Toronto, M4A 2X7 (CA)
(74) Representative: Pfenning, Meinig & Partner mbB

(56) References cited:
- WO-A1-2011/146243
- US-A- 5 221 281
- US-A1- 2006 178 646
- US-A1- 2006 224 156

## Description

### TECHNICAL FIELD

The disclosure relates to devices usable to deliver energy within the body of a patient. More specifically, the present disclosure is concerned with an electrosurgical perforation apparatus.

### BACKGROUND

WO 2011/146243 A1 discloses systems and methods for tissue ablation. Systems include needles with deployable filaments capable of producing asymmetrical offset lesions at target volumes, which may include a target nerve. Ablation of at least a portion of the target nerve may inhibit the ability of the nerve to transmit signals, such as pain signals, to the central nervous system. The offset lesion may facilitate procedures by directing energy towards the target nerve and away from collateral structures. Example anatomical structures include lumbar, thoracic, and cervical medial branch nerves and rami and the sacroiliac joint.

US 5,221,281 discloses an electrosurgical tubular trocar system that has a hollow tube substantially longer than its diameter. The tube is shaped for insertion in a direction generally along its axis through tissue of a human or animal body. Distal and proximal ends on the tube enter and remain outside the tissue, respectively. A tip on the distal end punctures tissue of a human or animal. An insulating portion of high dielectric material extends along the tube between the distal and proximal ends. An electrode on the insulating portion extends from the proximal end to the tip to transmit radio frequency energy. An energy supply at the electrode proximal end permits the passage of energy to the tip. An electrosurgical generator as part of the energy supply has a control to regulate the amplitude and frequency of the energy. A return path in circuit with the tip and the energy supply cuts and/or coagulates. A tip point at an acute angle to the axis lessens the initial force necessary for entry of the tube. The return path is a conductor on the insulating portion for bipolar cutting across a gap. A passage is made through the tissue. The tube may be conductive. The insulating portion may extend along the tube and be tubular. The electrode may be part of the tube when the conductor is on the insulating portion or the conductor may be part of the tube and the electrode may be on the insulating portion. The tube may be tapered from a smaller diameter at the tip and be smooth. The tip is chamfer and circular. An alternate system may have the return path as a conductive pad in contact with the tissue as a monopolar circuit. The tube may be in fluid communication for flow. A method of placing a trocar aligns an axis normal to the skin, energizes a generator, cuts electrosurgically tissue, drives the tube through the tissue, and disconnects the generator.

### SUMMARY

The invention is defined in the appended claims.

Disclosed herein are examples of an energy delivery device providing forward fluid delivery, while avoiding coring, the device comprising a distal face defining an opening, the distal face including at least one electrically exposed portion and at least one electrically insulated portion.

In one broad aspect, examples of the present disclosure include an energy delivery device comprising: an elongate member defining a lumen for receiving a fluid, a distal face of the elongate member defining an aperture in communication with the lumen, the distal face including at least one electrically exposed conductive portion and at least one electrically insulated portion, the distal face being thereby configured to avoid creation of emboli upon delivery of energy via the electrically exposed conductive portion.

As a feature of this aspect, some examples include the elongate member comprising an electrically conductive tubular member at least partially covered by electrically insulating material. Other examples include the elongate member comprising a non-conductive material.

For facilitating understanding of the invention, examples of the present disclosure show a method of puncturing tissue comprising the steps of: (a) delivering energy through a first portion of a distal face of an energy delivery device to tissue at a target site to create a puncture through the tissue, while preventing delivery of energy from a second portion of the distal face; and (b) advancing the energy delivery device through the tissue by pushing aside a flap of tissue defined by the puncture.

Another broad aspect of the disclosure is for an energy delivery device comprising: an electrically conductive tubular member at least partially covered by electrically insulating material, the tubular member defining a lumen for receiving a fluid, a distal face of the tubular member defining an aperture in communication with the lumen, and the distal face including at least one electrically exposed conductive portion and at least one electrically insulated portion, whereby energy delivered to a tissue via the at least one electrically exposed conductive portion results in a puncture having a shape substantially corresponding to a shape of the electrically conductive portion of the distal face.

In another broad aspect, examples of the disclosure are for an energy delivery device comprising: an elongate member defining a lumen for receiving a fluid, a beveled distal face of the elongate member defining an aperture in communication with the lumen and the beveled distal face including at least one electrically exposed conductive portion and at least one electrically insulated portion.

In yet another broad aspect examples of the disclosure are for an energy delivery device comprising: an elongate member defining a lumen for receiving a fluid; and a distal face defining an aperture in communication with the lumen, the distal face including at least one electrically exposed conductive portion and at least one electrically insulated portion; the distal face being configured to puncture through a tissue at a target site upon delivery of energy via the electrically exposed conductive portion substantially without coring tissue from the target site.

### BRIEF DESCRIPTION OF THE DRAWINGS

In order that the disclosure may be readily understood, examples of the disclosure are illustrated by way of examples in the accompanying drawings, in which:
Fig. 1 is an illustration of an example of a device including a handle and shaft;
Fig. 2 is an illustration of an electrically conductive tubular member with an angled front face;
Figs. 3a-c are illustrations of an example of a device with an electrically conductive tubular member and insulation;
Fig. 4 is an illustration of an alternative example in which an electrically conductive tubular member is comprised of proximal and distal components;
Figs. 5a to 5c are illustrations of another alternative example in which an electrically conductive tubular member receives a non-conductive insert and insulation;
Fig. 6 is an illustration of an example of a device with a non-conductive elongate member;
Figs. 7a and 7b are illustrations of an example of a method of puncturing tissue within a heart;
Fig. 8 is an example of a handle; and
Fig. 9a and 9b illustrates examples of embedded markers.

### DETAILED DESCRIPTION

Devices used for puncturing tissue, for example transseptal tissue of a patient's heart, are typically either mechanical or electrosurgical in nature. Some electrosurgical devices incorporate side-ports and do not have a forward facing lumen aperture, and consequently lack the ability, for example, to effectively inject fluid or monitor fluid pressure when confined inside of a close-fitting dilator lumen. In addition, while it is possible in some cases for a guide-wire to be passed through or to be received by a side-port, in general, devices lacking a forward facing aperture do not facilitate the use of a guide-wire with the device. In contrast, devices with a forward facing aperture typically are more effective in injecting fluid, monitoring pressure, and facilitate usage of a guide-wire.

A conventional Brockenbrough transseptal needle with a sharp beveled tip has a forward facing aperture that may be used for injecting fluid or monitoring pressure. However, conventional transseptal needles typically utilize mechanical force to puncture tissue, which is not effective at puncturing tissue under certain circumstances. To meet the challenge of puncturing through a tissue that does not facilitate being mechanically punctured, physicians have proposed using an electrocautery generator or the like to electrify the mechanical needle and to thereby produce an ad hoc electrosurgical device with a forward facing aperture. One drawback to electrifying a Brockenbrough needle is the risk of tissue coring. A core (or plug) of tissue is typically cut from surrounding tissue upon delivery of energy and is subsequently captured in the lumen of the electrosurgical device upon advancement of the needle through tissue. The tissue core may be released from the lumen by flushing, potentially leading to emboli and increasing the risk of a stroke or some other ischemic event. Furthermore, a non-insulated and electrified Brockenbrough needle bears an additional increased risk of burns to the patient and physician.

This disclosure includes different examples of a device that has a forward facing lumen aperture to provide for pressure monitoring, forward fluid delivery, being used with a guide-wire, and that is configured to reduce the risks of tissue coring and emboli formation.

In particular, the described examples are configured such that an electrode used for puncturing tissue does not completely encircle or enclose a forward facing lumen aperture, thereby avoiding having a ring-shaped electrode that may possibly core tissue. Ring-shaped electrodes are typically circular, but non-circular ringed electrodes (e.g. square-shaped, elliptical-shaped) are also possible. For explanatory purposes, this disclosure generally describes ring-shaped electrodes as being circular. Furthermore, while this disclosure describes electrosurgical devices that are generally circular in cross-section, the concepts and claims of this disclosure also apply to non-circular devices. Some examples comprise a device having a metallic tube that is operable to deliver energy in which a portion of the tube's distal (forward facing) face or surface is insulated to thereby interrupt the creation of a ring-shaped electrode such that the electrode only partially encircles the forward facing lumen aperture (or front opening). In some examples, the distal surface of the electrode (the cutting surface) is situated to be substantially lateral to the front opening, such that the electrode does not encircle the front opening. In typical examples, the outer perimeter of the electrode defines a portion, but not all, of the outer perimeter of the distal face of the of the disclosed energy device, whereby the device creates a puncture corresponding with a portion (but not all) of the outer perimeter of the distal face with the puncture defining a flap of tissue which the beveled distal face pushes aside when the device is advanced. In some examples, the distal surface of the electrode defines a shape selected from the group consisting of generally C-shaped shapes and generally semicircular shapes.

Thus, the present inventors have conceived and reduced to practice a needle for puncturing tissue, such as an atrial septum of a heart, where the needle allows for forward fluid delivery for staining the septum and has less risk of coring tissue relative to an electrified Brockenbrough needle. The device comprises a distal face defining an opening with the distal face including an electrically exposed portion and an electrically insulated portion extending radially from the opening to the periphery of the distal face. Typical examples can be advanced over a guide-wire to a treatment site.

With specific reference now to the drawings in detail, it is stressed that the particulars shown are by way of example and for purposes of illustrative discussion of certain examples of the present disclosure only. Before explaining examples of the disclosure in detail, it is to be understood that the disclosure is not limited in its application to the details of construction and the arrangement of the components set forth in the following description or illustrated in the drawings. The disclosure is capable of other examples or of being practiced or carried out in various ways. Also, it is to be understood that the phraseology and terminology employed herein is for the purpose of description and should not be regarded as limiting.

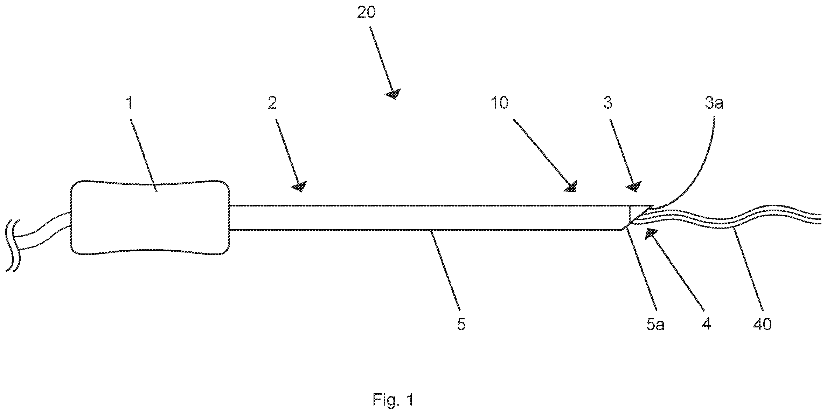

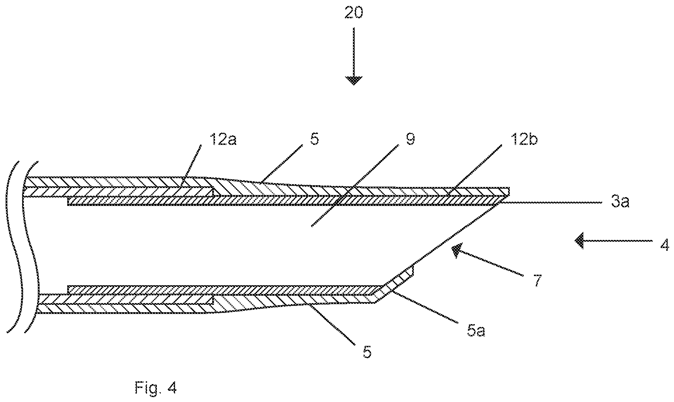

Fig. 1 is an illustration of an example of a device including a handle and shaft. Fig. 1 includes energy delivery device 20 which is comprised of elongate member 2, electrically insulating material 5 and distal portion 10. The handle 1 is mechanically coupled to the proximal end of the elongate member 2. Elongate member 2 defines a lumen (Fig. 4). Distal portion 10 includes electrode 3 and distal face 4 (further described herein below) which defines an aperture. The example is operable to direct a fluid forward, as represented by fluid flow lines 40, and facilitates being used with a guide-wire.

In some examples, elongate member 2 has a length of about 30 cm to about 100 cm to facilitate the puncture of a septum of a heart. In some examples, the elongate member has an outer diameter of about 0.40 mm to about 1.5 mm to minimize hemodynamic stability, for example, by ensuring that the perforation will not cause hemodynamic instability once energy delivery device 20 is removed. In some examples, the energy delivery device 20 is a stiff elongate needle.

Some examples of energy delivery device 20 include an elongate member 2 having flexural rigidity of at least about 0.016 Nm², for example a flexural rigidity of about 0.017 Nm², to provide tactile feedback for a user of the device.

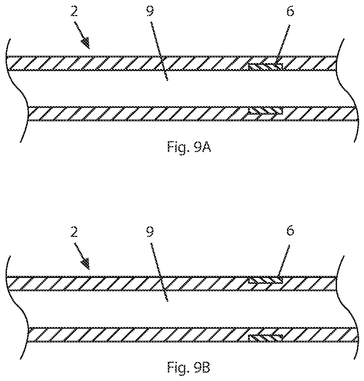

Some examples of the device have markers for highlighting the location of important landmarks on energy delivery device 20. Such landmarks may include the location where the elongated member 2 begins to curve, the location of the electrode 3, or the location of the proximal edge a beveled distal face. In some examples the marker is radiopaque. Fig. 9a and 9b illustrates examples of markers 6 embedded in the sidewall of elongate member 2. Fig. 9a is an example of an example in which a marker 6 is embedded in the inside surface of the sidewall of elongate member 2 whereby fluid can flow through lumen 9 without being obstructed and the outer diameter of elongate member 2 is not increased. Marker 6 can be embedded in the inside sidewall using different techniques, such as overmoulding. Fig. 9b is an example of an example in which a marker 6 is embedded in the outside sidewall of elongate member 2 whereby fluid can flow through lumen 9 without being obstructed and the outer diameter of elongate member 2 is not increased. Imaging marker 6 can be different shapes including, but not limited to, a ring-shaped hollow band or a coil. Alternative examples include imaging markers that are disc-shaped, rectangular, and elongate, that define other geometric shapes, or that define symbols.

For the examples of Figs. 9a and 9b, elongate member 2 can be comprised of one or more layers/components of plastic, other polymers, metal, or other materials. The marker is embedded in a sidewall which may be either all metal or substantially (mostly) metal. For example, the marker receiving sidewall can be covered with a relatively thin layer of polymer, such as the sidewall of Fig. 9b being covered with a layer of electrical insulation. As all metals are radiopaque to some degree, a radiopaque marker should be more radiopaque than the metal tube to function properly. In general, for any example of the device having a radiopaque marker, the radiopaque marker may be comprised of a material that is more radiopaque than whatever material elongate member 2 is comprised of. In Figs. 9a and 9b, the distal end of lumen 9 is open. Examples of Figs. 9a and 9b having a metal shaft can optionally have a layer of electrically insulating material 5 (not shown in Fig. 9).

While the example of Fig. 1 has a generally straight elongate member 2, in alternative examples, the elongate member comprises a curved section. In some examples, the curved section has a curve length of from about 10 to about 25 cm and traverses from about 20° to about 40° of a circle. In some other examples, the curved section has a curve length of from about 4 to about 7 cm and traverses from about 70 degrees to about 110 degrees of a circle.

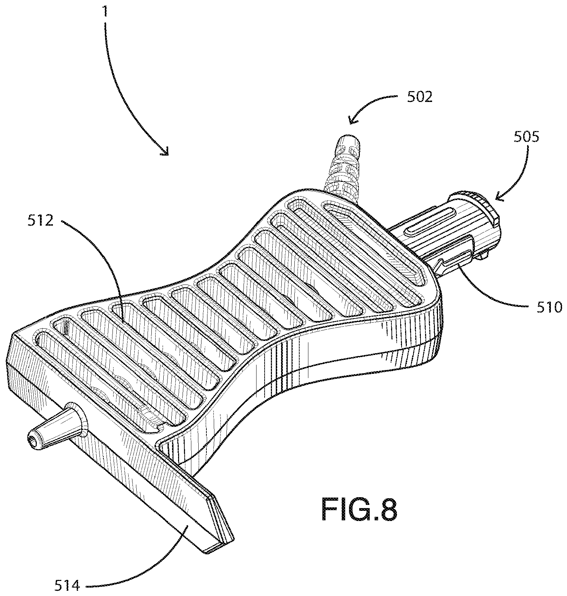

Fig. 8 illustrates an exemplary handle 1. Handle 1 comprises connector 502 for receiving an electrical plug or other electrical connector, and fluid port 505 for receiving a second connector, for example, a luer lock. Electrical energy may be delivered from an energy source, through a connector 502 and, typically, a wire (not shown in the drawing) located within handle 1. The electrical energy is then conveyed to the elongate member 2 and electrode 3.

In the example of Fig. 8, the handle 1 includes a relatively large graspable surface having ridges 512 so that tactile feedback can be transmitted relatively efficiently, for example by transmitting vibrations.

The example of Fig. 8 further comprises an orientation indicator 510 located on one side of the handle, in particular, on the side of the part of the handle housing fluid port 505. Orientation indicator 510 indicates the direction of the curve of the curved section of elongate member 2. The direction of the curve is also indicated by flange 514. In alternative examples, orientation indicators comprise inks, etching, or other materials that enhance visualization or tactile sensation.

In some examples, a tubing is operatively coupled at one end thereof to a source of fluid (not shown in drawing), for example a syringe, pump, intravenous fluid bag, etc., and the other end of the tubing is operatively coupled with a connector to fluid port 505 (of handle 1) which is in fluid communication with lumen 9 of elongate member 2 via a conduit in the handle (not shown), whereby the tubing and lumen 9 are in fluid communication with one another, thus allowing for a flow of fluid between an external device and lumen 9.

In some examples, aperture 7 and the lumen 9 (Figs. 4) together provide a pressure transmitting lumen which is coupled to the external tubing by a connector, and the tubing is in fluid communication with a pressure sensing device, for example a pressure transducer.

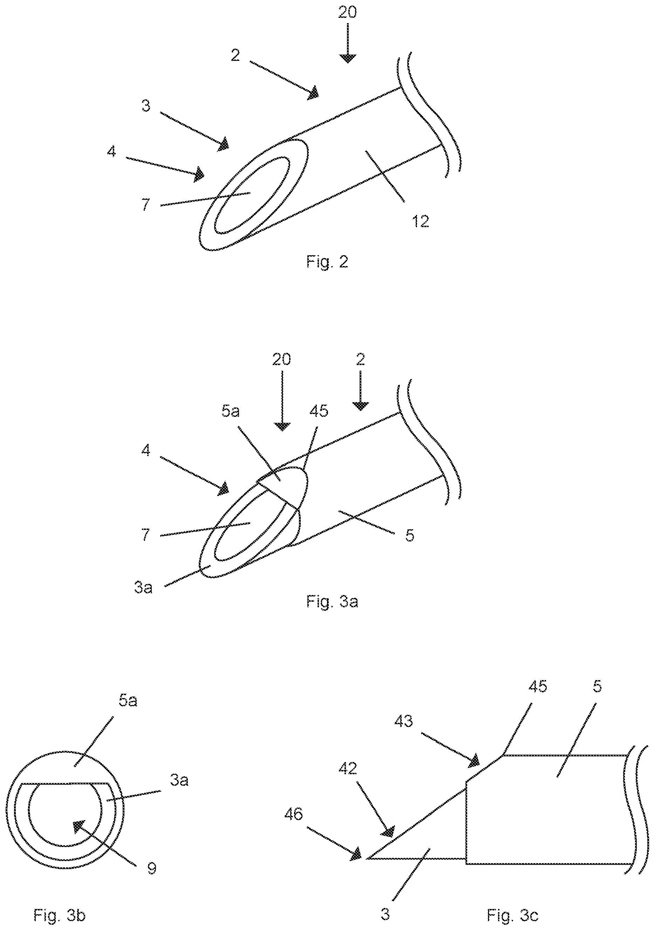

Referring now to Figure 2, Fig. 2 is an illustration of an example of an electrosurgical device initially lacking electrical insulation over its distal face. The device includes an electrically conductive tubular member 12 with an angled or beveled front face (distal face 4). As previously described, the distal (or front) surface of a device configured as illustrated in Fig. 2 may function as a ring-shaped electrode when advanced through tissue while delivering electrical energy, thereby coring out a plug of tissue having a circumference defined by aperture 7. Configuring a portion of the distal surface to be non-conductive may reduce or eliminate the risk of coring tissue, as described further hereinbelow.

Figs. 3a to 3c illustrate the distal portion of an example of an energy delivery device 20 having an electrically conductive tubular member. Energy delivery device 20 comprises an elongate member 2 which defines a lumen 9 for receiving a fluid and a distal face 4 which defines an aperture 7 in communication with the lumen. The fluid within the lumen may be injected, withdrawn, or may remain substantially stationary. The electrically conductive tubular member is at least partially covered by electrically insulating material 5 with a distal portion of the electrically conductive tubular member uncovered (i.e. electrically exposed) to define electrode 3. In some examples, the electrically conductive tubular member is comprised of stainless steel.

In the example of Fig. 3, distal face 4 is beveled and is comprised of an electrically exposed conductive portion 3a and an electrically insulated portion 5a. The electrically exposed conductive portion 3a is comprised of the distal surface of electrode 3 which, in this example, is generally C-shaped when viewing the distal face head-on. The electrically exposed conductive portion 3a does not completely encircle, circumscribe or enclose aperture 7 but rather partially surrounds the aperture. A distal portion of the distal face 4 comprises a distal surface of electrode 3 (i.e. the beveled face is not truncated).

The proximal portion of distal face 4 is comprised of electrically insulated portion 5a. Electrically insulated portion 5a extends radially from a wall of the aperture 7 to a periphery 45 of distal face 4 (i.e. extending from the center outwardly or from the circumference inwardly). In some examples, the electrically insulated portion of distal face 4 is comprised of polymer insulation, which may be a heat shrink, a spray coating, or a material selectively coated by vapor deposition. In some alternative examples, electrically insulated portion 5a comprises a ceramic. In some examples, the distal face of the electrically conductive tubular member has a step recess wherein a layer of insulation is received to thereby provide for a planar distal face 4 (i.e. to avoid having a stepped surface).

The electrically exposed conductive portion 3a is configured such that, when the energy delivery device is advanced into a tissue, energy delivered by the electrically exposed conductive portion 3a punctures the tissue without the tissue substantially occluding lumen 9. In particular, it is the leading surface of electrode 3 that defines the cutting surface of the electrode (i.e. electrically exposed conductive portion 3a) which actually cuts into tissue when the energy delivery device is advanced while delivering energy. Electrically exposed conductive portion 3a does not completely encircle the forward facing lumen aperture 7, thereby avoiding having a ring-shaped (i.e. closed-loop, when viewed head-on) electrode that could core tissue. Electrically insulated portion 5a interrupts the distal surface of electrically conductive tubular member 12 to prevent the formation of a ring-shaped electrode such that the cutting surface of the electrode only partially encircles aperture 7. The outer perimeter of the distal surface of electrode 3 defines a portion (but not all) of the perimeter of distal face 4, whereby the device creates a puncture corresponding with a portion (but not all) of the perimeter of the distal face, such that the puncture defines a flap of tissue which the beveled distal face pushes aside as the device is advanced.

The example of energy delivery device 20 of Fig. 3 includes a distal tip 46 which is substantially rounded or atraumatic, as it is not necessary to have a sharp tip on the device for puncturing. The rounded tip reduces the risk of accidental tissue puncture and skiving of supporting dilators. In other words, the distal portion of the distal face is substantially rounded. In some alternative examples, the tip of the device is sharp. Furthermore, the planar surface of distal face 4 is substantially atraumatic.

Fig. 4 illustrates another example of an energy delivery device 20 having an electrically conductive tubular member. The example of Fig. 4 includes the electrically conductive tubular member being comprised of two parts, a proximal electrically conductive tubular member 12a, and a distal electrically conductive tubular member 12b which fits into the proximal tubular member. The two tubular members are joined together in any suitable manner, for example welding, soldering, friction fitting, or the use of adhesives.

This example also includes electrically insulating material 5 extending to the end of the outer surface of distal electrically conductive tubular member 12b to help minimize electrical leakage. Electrically insulating material 5 extends to cover a portion on the distal end surface of electrically conductive tubular member 12b to define electrically insulated portion 5a of distal face 4 (seen in the bottom of the drawing). In some alternative examples, electrically insulated portion 5a is comprised of a different material than the insulating material 5 which covers the sidewalls of electrically conductive tubular members 12a and 12b.

Similar to the example of Fig. 3, the example of Fig. 4 has an electrically exposed conductive portion 3a which is generally C-shaped. The example of Fig. 4 functions in substantially the same manner as the example of Fig. 3.

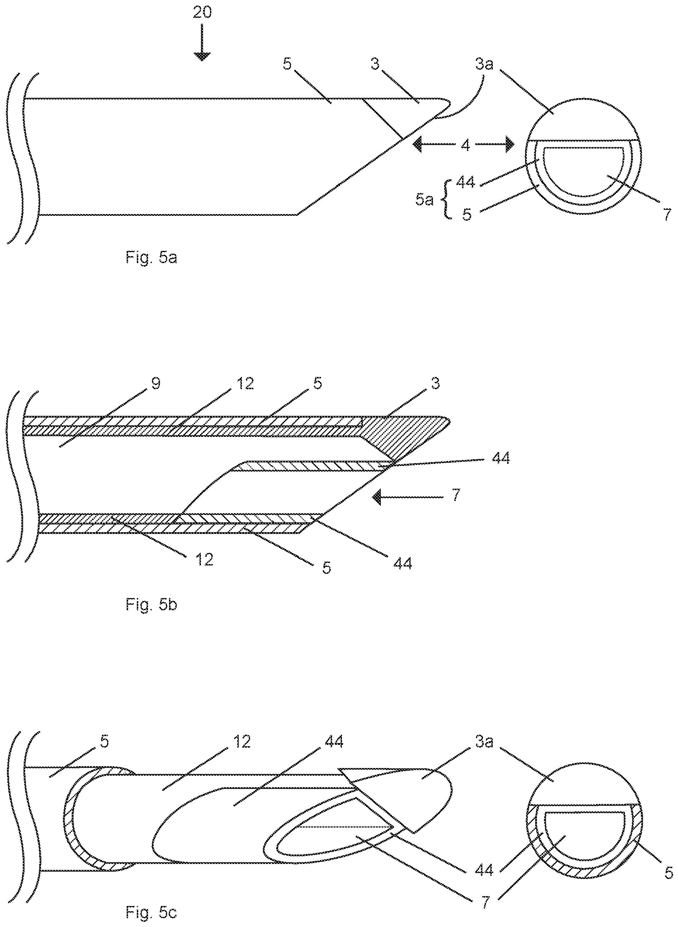

Figs. 5a to 5c illustrate another example of energy delivery device 20 with an electrically conductive tubular member. The energy delivery device of Fig 5 includes a recess cut away from the end of electrically conductive tubular member 12 for receiving non-conductive insert 44. Typically, non-conductive insert 44 is a polymer. In some examples, non-conductive insert 44 is a stiff plastic, and in some particular examples is re-flowed FEP (Fluorinated ethylene propylene). Fig. 5c, which is a rotated side-view, illustrates the device with electrically insulating material 5 partially cut away and shows how electrically conductive tubular member 12 receives non-conductive insert 44.

Fig. 5b is a cut-away side-view illustrating that electrode 3 extends from electrically conductive tubular member 12. The side-view of Fig. 5a and Fig. 5b show that electrode 3 is an electrically exposed portion of tubular member 12 i.e. the electrode is continuous with conductive tubular member 12 and is not covered by electrically insulating material 5.

The end view of Fig. 5a shows the non-conductive insert 44 located between a layer of electrically insulating material 5 and electrode 3. Figs. 5b and 5c show that non-conductive insert 44 fits into the recess in electrically conductive tubular member 12, and that insulating material 5 encloses both conductive insert 44 and electrically conductive tubular member 12.

As seen in the Fig. 5a end-view, the electrically insulated portion 5a of distal face 4 is comprised of the end surfaces of both electrically insulating material 5 and non-conductive insert 44. Electrically exposed conductive portion 3a is comprised of the distal surface of electrode 3. The end views of Fig. 5 show that electrically exposed conductive portion 3a is substantially semi-circular in shape and that electrically insulated portion 5a extends radially from aperture 7 to the periphery 45 of the distal face 4. Electrically exposed conductive portion 3a does not fully or partially encircle aperture 7, but instead is lateral to aperture 7, and consequently does not form a ring-shaped electrode capable of coring out tissue.

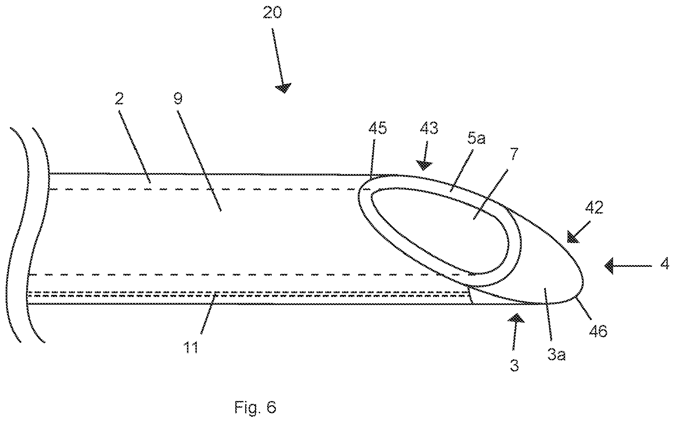

Fig. 6 illustrates an example of energy delivery device 20 having a non-conductive elongate member 2. Electrode 3 is attached to the end of the elongate member. Typically, a non-conductive elongate member 2 is comprised of polymer.

The example of Fig. 6 has a beveled distal face 4. The electrically exposed conductive portion 3a of distal face 4 comprises a distal surface of electrode 3 and is generally crescent-shaped. Wire 11 is connected to electrode 3 for delivering energy thereto. In typical examples, wire 11 is contained in a lumen of appropriate size. Alternatively, wire 11 is embedded in a wall of the non-conductive elongate member 2.

In the illustrated example, the electrically insulated portion 5a is located at a proximal portion of distal face 4 and is comprised of the distal surface of the non-conductive elongate member 2. When the example of Fig. 6 is viewed from the end, electrically insulated portion 5a encircles aperture 7, and electrically exposed conductive portion 3a does not encircle the aperture, but instead is lateral to the aperture, and consequently does not form a ring-shaped electrode capable of coring tissue.

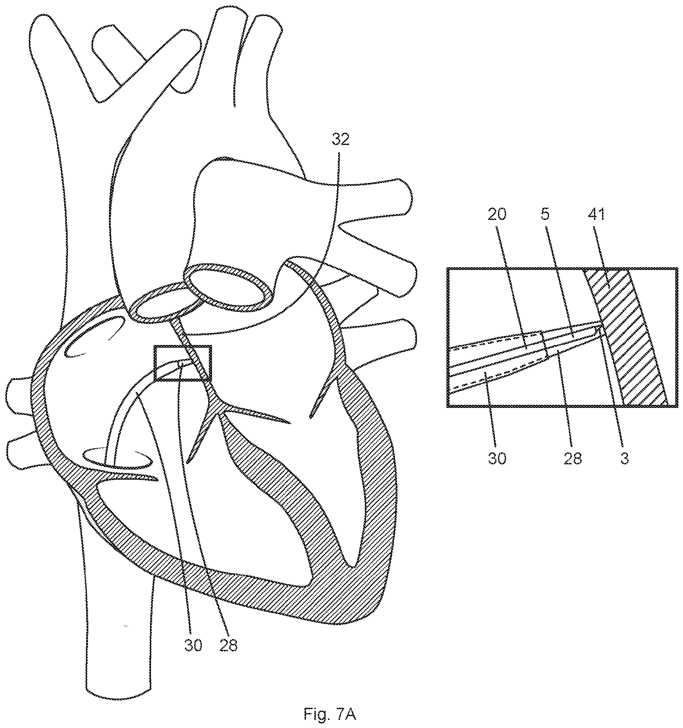

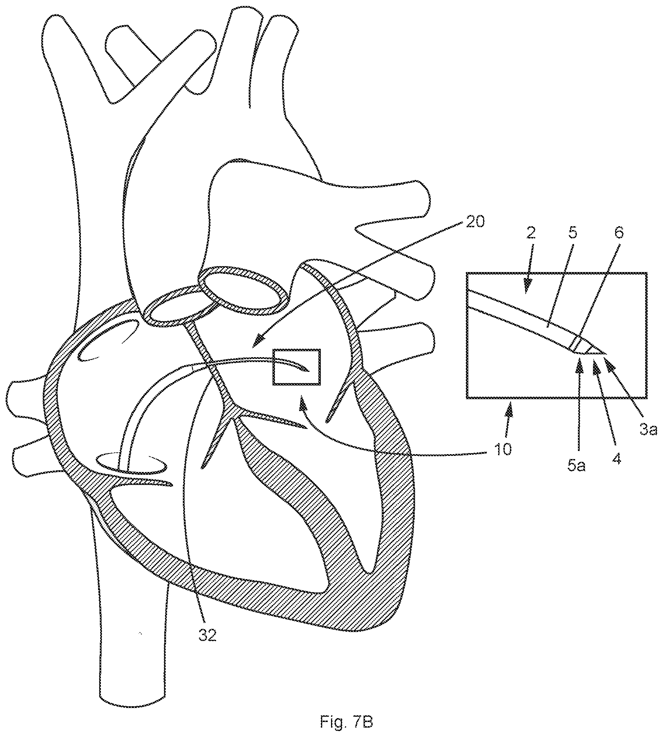

Figs. 7a and 7b illustrate an example of a method of puncturing tissue. The method comprises the steps of (a) delivering energy through electrically exposed conductive portion 3a of energy delivery device 20 to tissue 41 at a target site for creating a puncture substantially corresponding to a distal surface of the electrically exposed conductive portion; and (b) dilating the puncture primarily by advancing an angled distal surface of the energy delivery device, without coring the tissue. In some examples the step of delivering energy comprises creating a flap in the tissue and the step of dilating is completed without further delivery of energy. In some examples, the target site is a tissue within a heart, and in some particular examples the tissue is an atrial septum 32. Typically, the method uses a sheath, such as, for example, sheath 30 of Fig. 7a.

An alternative example of a method of puncturing tissue comprises the steps of (a) delivering energy through a first portion of a distal face of an energy delivery device to tissue at a target site to create a puncture through the tissue, while preventing delivery of energy from a second portion of the distal face; and (b) advancing the energy delivery device through the tissue by pushing aside a flap of tissue defined by the puncture. The step of delivering energy comprises creating a slit in the tissue.

Dilating the puncture typically includes displacing the tissue. In some examples dilation includes wedging apart and thereby outwardly compressing surrounding portions of the tissue.

Some examples of the method include using a medical imaging modality to guide the energy delivery device 20 to the target site. Some examples comprise measuring pressure for positioning energy delivery device at the target site. In some examples, the method includes using a radiopaque marker 6 for positioning energy delivery device 20. Some examples include advancing the energy delivery device to the target site over a guide-wire.

In some examples, the method includes advancing energy delivery device 20 to the target site through a dilator 28; positioning energy delivery device 20 such that electrically exposed conductive portion 3a is aligned with or protruding slightly from a distal end of the dilator 28; and delivering fluid through an aperture (e.g. Fig. 3) at a distal end of energy delivery device 20 to stain the tissue. The fluid is typically delivered longitudinally forward through the energy delivery device. Some examples further comprise a step of withdrawing a fluid via an open distal face of the energy delivery device.

In some examples, the distal surface of the electrically exposed conductive portion 3a is generally C-shaped and step (b) includes creating a generally C-shaped puncture. In some other examples, the distal surface of the electrically exposed conductive portion is generally crescent-shaped and step (b) includes creating a generally crescent-shaped puncture.

In some examples of the broad aspect, the aperture 7 and the lumen 9 together comprise a pressure transmitting lumen, and the method further comprises measuring a fluid pressure of the pressure transmitting lumen using a pressure sensing mechanism.

In an RF perforation or puncturing procedure, unlike RF ablation, energy is applied to rapidly increase tissue temperature to the extent that the intracellular fluid becomes converted to steam, inducing cell lysis as a result of elevated pressure within the cell. Upon the occurrence of cell lysis and rupture, a void is created, allowing the tip of the catheter to penetrate the tissue. In order to achieve this effect, RF perforation devices must apply a high voltage to the tissue region over a short period of time. Also, the tip of the device being used should be relatively small, in order to increase the impedance of the device. This is in contrast to RF ablation, whereby a larger-tipped device is utilized to deliver a low impedance and high power signal to the region involved. Furthermore, as opposed to RF perforation, which creates a void in the tissue through which the device may be advanced, the objective of RF ablation is to create a large, non-penetrating lesion in the tissue, in order to disrupt electrical conduction. Thus, for the purposes of the present disclosure, perforation is defined as the creation of a void within a material.

Examples of the present disclosure are operable to create such punctures or voids without substantially removing a plug or core of material from the tissue at the target site, since the puncture resulting from devices as described hereinabove are typically slit-like, C-shaped, or similar configurations corresponding to the shape(s) of the electrically exposed and conductive portion of the distal face.

Energy delivery device 20 may be used in conjunction with a source of radiofrequency energy suitable for perforating material within a patient's body. The source of energy may be a radiofrequency (RF) electrical generator, operable in the range of about 100 kHz to about 1000 kHz, and designed to generate a high voltage over a short period of time. More specifically, in some examples, the voltage generated by the generator increases from about 0 V (peak-to-peak) to greater than about 75 V (peak-to-peak) in less than about 0.6 seconds. The maximum voltage generated by generator may be between about 180V peak-to-peak and about 3000V peak-to-peak. The waveform generated may vary, and may include, for example, a sine-wave, a rectangular-wave, or a pulsed rectangular wave, amongst others. During delivery of radiofrequency energy, the impedance load may increase due to tissue lesioning near the target-site, or the formation of a vapor layer following cell rupture, for example. The generator may be operable to continue to increase the voltage, even as the impedance load increases. For example, energy may be delivered to a tissue within a body at a voltage that rapidly increases from about 0 V (RMS) to about 220 V (RMS) for a period of between about 0.5 seconds and about 5 seconds.

Without being limited to a particular theory of operation, it is believed that under particular circumstances, for example as mentioned hereinabove, dielectric breakdown and arcing may occur upon the delivery of radiofrequency energy, whereby polar molecules may be pulled apart. The combination of these factors may result in the creation of an insulative vapor layer around the electrode, therein resulting in an increase in impedance, for example the impedance may increase to greater than 4000 Ω. In some examples, despite this high impedance, the voltage continues to increase. Further increasing the voltage increases the intensity of fulguration, which may be desirable as it allows for an increased perforation rate and puncture creation. An example of an appropriate generator for this application is the BMC RF Perforation Generator (model number RFP-100A, Baylis Medical Company, Montreal, Canada). This generator delivers continuous RF energy at about 460 kHz.

A grounding pad or dispersive electrode may be electrically coupled to the generator for contacting or attaching to the body of the patient to provide a return path for the RF energy when the generator is operated in a monopolar mode.

Additional details regarding the device and method may be found in U.S. application Ser. No. 13/468,939, filed May 10, 2012, U.S. application Ser. No. 11/905,447, filed Oct. 1, 2007 (now issued as U.S. patent 8,192,425), U.S. application Ser. No. 13/113,326, filed May 23, 2007, U.S. application Ser. No. 11/265,304, filed Nov. 3, 2005 (now U.S. patent 7,947,040), U.S. application Ser. No. 10/666,301, filed Sep. 19, 2003 (now issued as U.S. patent 7,048,733), U.S. application Ser. No. 10/760,479, filed Jan. 21, 2004 (now issued as U.S. patent 7,270,662), U.S. application Ser. No. 10/666,288, filed Sep. 19, 2003, U.S. application Ser. No. 10/347,366, filed Jan. 21, 2003 (now issued as U.S. patent 7,112,197), U.S. provisional application Ser. No. 60/522,753, filed Nov. 3, 2004, and provisional applications Ser. No. 60/884,285, filed Jan. 10, 2007, 60/827,452, filed Sep. 29, 2006, Ser. No. 61/653967, filed May 31, 2012, and Ser. No. 61/681,512, filed Aug. 9, 2012.

Thus, the problem of puncturing tissue without coring, while providing forward fluid delivery, is solved by an energy delivery device having a distal face defining an opening, with the distal face including at least one electrically exposed portion and at least one electrically insulated portion.

### Example 1:

Examples having the configuration of Fig. 3 were tested and found to puncture tissue substantially without coring. Electrified Brockenbrough needles were also tested, and found to core tissue when puncturing. The testing revealed that Fig. 3 examples cut C-shaped punctures that correspond to the shape of the electrode when viewed from the end, resulting in a flap of skin that is displaced sideways by the proximal portion of distal face 4 when energy delivery device 20 is advanced, whereby the C-shaped puncture is dilated.

The examples of the disclosure described above are intended to be exemplary only. The scope of the invention is therefore intended to be limited solely by the scope of the appended claims.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art.
Accordingly, it is intended to embrace all such alternatives, modifications and variations that fall within the broad scope of the appended claims. In addition, citation or identification of any reference in this application shall not be construed as an admission that such reference is available as prior art to the present invention.

## Claims

1. An energy delivery device (20) comprising:
an elongate member (2) which comprises a non-conductive material and defines a lumen for receiving a fluid, a distal face (4) of the elongate member (2) defining an aperture (7) in communication with the lumen, wherein all of the distal face (4) defines a beveled surface, and
the beveled surface of the distal face (4) consisting of a single electrically exposed
conductive portion (3a) and a single electrically insulated portion (5a), wherein the single
electrically exposed conductive portion (3a) is lateral to the aperture (7) and the single electrically insulated portion (5a) extends radially from a wall of the aperture (7) to a periphery of the distal face (4) and encircles the aperture,
wherein the single electrically exposed conductive portion (3a) comprises a distal surface of a single electrode located at a furthest distal portion of the beveled surface,
the distal face (4) being thereby configured to avoid creation of emboli upon delivery of energy via the single electrically exposed conductive portion (3a).

2. The energy delivery device (20) of claim 1, wherein all of the single electrically insulated portion (5a) is proximal of at least some of the single electrically exposed conductive portion (3a).

3. The energy delivery device (20) of claim 1, wherein the distal surface of the single electrode (3) is generally crescent-shaped.

4. The energy delivery device (20) of claim 1, further comprising a wire which is electrically coupled to the single electrode (3), the wire being operable to convey energy to the single electrode (3).

5. The energy delivery device (20) of claim 4, wherein the wire is embedded in a sidewall of the elongate member.

6. The energy delivery device (20) of claim 1, wherein the single electrically insulated portion (5a) comprises a polymer located at a proximal portion of the distal face (4).

7. The energy delivery device (20) of claim 1, wherein the single electrically insulated portion (5a) completely surrounds and defines the aperture (7) and the single electrically exposed conductive portion (3a) is lateral to and spaced apart from the aperture (7).

8. The energy delivery device (20) of claim 1, wherein the elongate member (2) has an outer diameter of about 0.40 mm to about 1.5 mm to minimize hemodynamic instability upon puncturing tissue.

9. The energy delivery device (20) of claim 1, wherein the elongate member (2) has a flexural rigidity of at least about 0.016 Nm².

10. The energy delivery device (20) of claim 9, wherein the elongate member (2) has a flexural rigidity of about 0.017 Nm².

11. The energy delivery device (20) of claim 1, further comprising
a radiopaque marker for marking a location of the electrode, wherein the radiopaque marker is embedded in a sidewall of the elongate member (2).

12. The energy delivery device (20) of claim 1, further comprising a handle (1) coupled to a proximal end of the elongate member (2).

13. The energy delivery device (20) of claim 12, wherein the handle (1) comprises an electrical connector operable to be coupled to an electrosurgical generator.

14. The energy delivery device of claim 12, wherein the handle (1) comprises a fluid port for allowing for fluid communication between the lumen and a source of fluid.

15. The energy delivery device of claim 1, wherein the elongate member (2) has a length of 30cm to 100cm to facilitate a puncture of a septum of a heart.

## Patentansprüche

1. Energieabgabevorrichtung (20), umfassend:
ein längliches Element (2), das ein nicht-leitendes Material umfasst und ein Lumen für eine Aufnahme eines Fluids definiert,
eine distale Fläche (4) des länglichen Elements (2), die eine Öffnung (7) in Verbindung mit dem Lumen definiert,
wobei die gesamte distale Fläche (4) eine abgeschrägte Oberfläche definiert und die abgeschrägte Oberfläche der distalen Fläche (4) aus einem einzelnen elektrisch freiliegenden leitenden Abschnitt (3a) und einem einzelnen elektrisch isolierten Abschnitt (5a) besteht,
wobei der einzelne elektrisch freiliegende leitende Abschnitt (3a) seitlich der Öffnung (7) liegt und der einzelne elektrisch isolierte Abschnitt (5a) sich radial von einer Wand der Öffnung (7) zu einem Umfang der distalen Fläche (4) erstreckt und die Öffnung umgibt,
wobei der einzelne elektrisch exponierte leitende Abschnitt (3a) eine distale Oberfläche einer einzelnen Elektrode umfasst, die an einem äußerst distalen Abschnitt der schrägen Oberfläche angeordnet ist,
wobei die distale Fläche (4) dadurch so konfiguriert ist, dass eine Erzeugung von Emboli bei einer Abgabe von Energie mittels des einzelnen elektrisch exponierten leitenden Abschnitts (3a) vermieden wird.

2. Energieabgabevorrichtung (20) nach Anspruch 1, wobei der einzelne elektrisch isolierte Abschnitt (5a) insgesamt proximal ist in Bezug auf zumindest einen Teil des einzelnen elektrisch exponierten leitenden Abschnitts (3a).

3. Energieabgabevorrichtung (20) nach Anspruch 1, wobei die distale Oberfläche der einzelnen Elektrode (3) allgemein sichelförmig ist.

4. Energieabgabevorrichtung (20) nach Anspruch 1, ferner einen Draht umfassend, der elektrisch mit der einzelnen Elektrode (3) gekoppelt ist, wobei der Draht dazu dient, Energie auf die einzelne Elektrode (3) zu übertragen.

5. Energieabgabevorrichtung (20) nach Anspruch 4, wobei der Draht in eine Seitenwand des länglichen Elements eingebettet ist.

6. Energieabgabevorrichtung (20) nach Anspruch 1, wobei der einzelne elektrisch isolierte Abschnitt (5a) ein Polymer umfasst, das an einem proximalen Abschnitt der distalen Fläche (4) angeordnet ist.

7. Energieabgabevorrichtung (20) nach Anspruch 1, wobei der einzelne elektrisch isolierte Abschnitt (5a) die Öffnung (7) vollständig umgibt und definiert und der einzelne elektrisch exponierte leitende Abschnitt (3a) in Bezug auf die Öffnung (7) lateral und beabstandet ist.

8. Energieabgabevorrichtung (20) nach Anspruch 1, wobei das längliche Element (2) einen Außendurchmesser von etwa 0,40 mm bis etwa 1,5 mm aufweist, um eine hämodynamische Instabilität bei einer Punktierung von Gewebe zu minimieren.

9. Energieabgabevorrichtung (20) nach Anspruch 1, wobei das längliche Element (2) eine Biegesteifigkeit von mindestens 0,016 Nm² aufweist.

10. Energieabgabevorrichtung (20) nach Anspruch 9, wobei das längliche Element (2) eine Biegesteifigkeit von mindestens 0,017 Nm² aufweist.

11. Energieabgabevorrichtung (20) nach Anspruch 1, ferner einen strahlenundurchlässigen Marker für eine Markierung einer Stelle, wo sich die Elektrode befindet, umfassend, wobei der strahlenundurchlässige Marker in eine Seitenwand des länglichen Elements (2) eingebettet ist.

12. Energieabgabevorrichtung (20) nach Anspruch 1, ferner einen Griff (1) umfassend, der mit einem proximalen Ende des länglichen Elements (2) gekoppelt ist.

13. Energieabgabevorrichtung (20) nach Anspruch 12, wobei der Griff (1) einen elektrischen Anschluss umfasst, der dazu dient, mit einem elektrochirurgischen Generator gekoppelt zu werden.

14. Energieabgabevorrichtung nach Anspruch 12, wobei der Griff (1) einen Fluiddurchlass umfasst, um eine Fluidverbindung zwischen dem Lumen und einer Fluidquelle zu ermöglichen.

15. Energieabgabevorrichtung nach Anspruch 1, wobei das längliche Element (2) eine Länge von 30 cm bis 100 cm aufweist, um eine Punktierung eines Septums eines Herzens zu ermöglichen.

## Revendications

1. Dispositif de distribution d'énergie (20) comprenant :
un élément allongé (2) constitué d'un matériau non conducteur et définissant une lumière destinée à recevoir un fluide,
une face distale (4) de l'élément allongé (2) définissant une ouverture (7) en communication avec la lumière,
dans lequel toute la face distale (4) définit une surface biseautée, la surface biseautée de la face distale (4) étant constituée d'une seule partie conductrice électriquement exposée (3a) et d'une seule partie électriquement isolée (5a),
dans lequel la partie conductrice unique électriquement exposée (3a) est latérale à l'ouverture (7) et la partie unique électriquement isolée (5a) s'étend radialement d'une paroi de l'ouverture (7) à une périphérie de la face distale (4) et encercle l'ouverture,
dans lequel la partie conductrice unique électriquement exposée (3a) comprend une surface distale d'une électrode unique située dans la partie distale la plus éloignée de la surface biseautée,
la face distale (4) est ainsi configurée pour éviter la création d'emboles lors de l'administration d'énergie par la seule partie conductrice électriquement exposée (3a).

2. Le dispositif de fourniture d'énergie (20) de la revendication 1, dans lequel toute la partie isolée électriquement (5a) est proximale d'au moins une partie de la partie conductrice exposée électriquement (3a).

3. Le dispositif d'administration d'énergie (20) de la revendication 1, dans lequel la surface distale de l'électrode unique (3) est généralement en forme de croissant.

4. Le dispositif de fourniture d'énergie (20) de la revendication 1, comprenant en outre un fil électriquement couplé à l'électrode unique (3), le fil étant capable d'acheminer de l'énergie à l'électrode unique (3).

5. Le dispositif d'administration d'énergie (20) de la revendication 4, dans lequel le fil est intégré dans une paroi latérale de l'élément allongé.

6. Le dispositif d'administration d'énergie (20) de la revendication 1, dans lequel la partie unique isolée électriquement (5a) comprend un polymère situé dans une partie proximale de la face distale (4).

7. Le dispositif de distribution d'énergie (20) de la revendication 1, dans lequel la partie isolée électriquement (5a) entoure et définit complètement l'ouverture (7) et la partie conductrice exposée électriquement (3a) est latérale et espacée de l'ouverture (7).

8. Le dispositif d'administration d'énergie (20) de la revendication 1, dans lequel l'élément allongé (2) a un diamètre extérieur d'environ 0,40 mm à environ 1,5 mm pour minimiser l'instabilité hémodynamique lors de la perforation du tissu.

9. Le dispositif d'administration d'énergie (20) de la revendication 1, dans lequel l'élément allongé (2) a une rigidité de flexion d'au moins environ 0,016 Nm².

10. Le dispositif d'administration d'énergie (20) de la revendication 9, dans lequel l'élément allongé (2) a une rigidité de flexion d'environ 0,017 Nm².

11. Le dispositif d'administration d'énergie (20) de la revendication 1, comprenant en outre un marqueur radio-opaque pour marquer l'emplacement de l'électrode, le marqueur radio-opaque étant encastré dans une paroi latérale de l'élément allongé (2).

12. Le dispositif d'administration d'énergie (20) de la revendication 1, comprenant en outre une poignée (1) couplée à une extrémité proximale de l'élément allongé (2).

13. Le dispositif d'administration d'énergie (20) de la revendication 12, dans lequel la poignée (1) comprend un connecteur électrique pouvant être couplé à un générateur électrochirurgical.

14. Le dispositif d'administration d'énergie de la revendication 12, dans lequel la poignée (1) comprend un port de fluide pour permettre la communication de fluide entre la lumière et une source de fluide.

15. Le dispositif d'administration d'énergie de la revendication 1, dans lequel l'élément allongé (2) a une longueur de 30 cm à 100 cm pour faciliter la perforation du septum d'un coeur.
